# EUROPEAN PATENT SPECIFICATION

(11) **EP 0 578 702 B1**
(45) Date of publication and mention of the grant of the patent: **24.01.1996**
(21) Application number: 92907821.0
(22) Date of filing: 31.03.1992
(51) Int. Cl.: A61K 7/48, A01N 31/02, A01N 35/06, A01N 37/02, A61K 7/40

(54) **AN AGENT FOR ANIMAL SKIN TREATMENT**
WIRKSTOFF ZUR HAUTBEHANDLUNG BEI TIEREN
AGENT DE TRAITEMENT DE LA PEAU D'ANIMAUX

(30) Priority: 09.04.1991 SE 9101056
(43) Date of publication of application: 19.01.1994
(73) Proprietor: AB KVARNBYFODER, S-245 00 Staffanstorp (SE)
(72) Inventor: EKELUND, Gert, S-222 48 Lund (SE)
(74) Representative: Onn, Thorsten
(86) International application number: SE9200203
(87) International publication number: WO9218099

(56) References cited:
- EP-A- 0 030 576
- GB-A- 1 588 649
- SE-B- 461 891

## Description

The present invention relates to an agent for the skin treatment of animals, preferably those parts of the skins of domestic animals, livestock, which are relatively free from hair, and then particularly for the treatment of cow udders, teats; and further relates to a method for the manufacture of said agent and also to the use of said agent.

It has long been endeavoured in the breeding of livestock and cattle to maintain the animals vigorous and in good health, and to prevent injury to the animals. It is particularly important that the skin of the animals is not subjected to swellings and infection which may have a deleterious effect on the actual skin itself and also constitute sites for injury and sickness to other parts of the animal's body.

In the case of dogs and cats, for instance, the thinly hair covered skin on the stomach of the animal is particularly subjected to injury as a result of scratching, dehydration, insect bites, etc. This also applies to other, larger domestic animals, such as horses and pigs, and to smaller domestic animals, such as guinea pigs, hamsters, etc.

The udders and teats of milk-producing cows are placed under particularly high strain, which quickly results in swellings and infection in the skin of these organs. This can, in turn, affect the milk produced, which may then contain excessively high concentrations of unpermitted bacteria, thereby rendering it necessary to discard the milk. Drugs are available commercially which will cure relatively quickly the majority of illnesses and sickness caused by swellings and infections. The economic loss caused by discarding the contaminated milk is often relatively high, however. Furthermore, cows suffering an udder infection may need to run dry and may be subjected to other illnesses as a result thereof. It is therefore extremely important to prevent the occurrence of swellings and infections, instead of curing the illnesses and injuries that occur.

Those preventative agents which are known in this regard, and particularly for the preventative treatment of cows, include ointments and other preparations, each of which is normally used to treat a particularly complaint. For example, camphor is used against swellings, and iodine is used for disinfection purposes. Swedish Patent Application 8604943-4 EP-A-0 290 572, family member teaches an agent which is used for treating several swelling and infection conditions simultaneously. This agent is comprised of a mixture of soft soap, peanut oil, spirits of camphor, vinegar essence, low-molecular aliphatic alcohol and water.

Although the agent taught by this patent application possesses some good properties, it is also encumbered with a number of drawbacks, of which the most serious is that the agent is unable to form a homogenous mixture and therefore quickly layers out, with the lighter peanut oil located on top of the heavier components. This means that the agent must be shaken before being applied to the udders or teats of the animal under treatment. In order to avoid the spread of infection, it is necessary to spray the agent onto the body of the animal under treatment, and if the agent is not shaken prior to being applied, practically pure peanut oil will be applied first to the cow's skin. Although this has a soothing effect, it does not have a disinfecting effect.

When the majority of the peanut oil has been applied, the aggressive components, such as spirits of camphor, alcohol and vinegar essence, lying beneath the peanut oil are sprayed directly onto the udders, with the risk of the teats being burned away. Obviously, it is quite easy to forget to shake the agent. The fact that the agent is not homogeneously mixed also means that the agent cannot be readily applied with the aid of a centralized spray system, i.e. a large storage container to which spray hoses are connected. Such a storage container must be provided with an agitator or stirring device or must be equipped with means for shaking the container, thereby rendering the process more expensive.

In order to be effective, it is essential that the agent remains on the skin for a sufficient length of time. The agent taught by the aforesaid Swedish patent application, however, does not adhere sufficiently to the udders and teats of cows, and readily runs off. The durability of the agent is also limited, since the peanut oil therein gradually becomes rancid. Durability of the agent can be extended solely by keeping the agent cool, which may be difficult to arrange in a simple manner.

The present invention therefore relates to an agent for the skin treatment of animals, preferably animal skin which is essentially free from hair, i.e. thinly-haired, and then particularly the udders and teats of cows, with which the aforedescribed drawbacks are avoided. This object is achieved with an agent which has complete and practically unchangeable homogeneity, which will adhere strongly to skin, and which has long durability, even in the absence of cooling.

The inventive agent is composed of a well-tested mixture of isopropanol, soft soap, propylene glycol, spirits of camphor, vinegar essence, cooking salt and water. Isopropanol is pure and undiluted (CH₃)₂CHOH. By soft soap is meant conventional yellow soap containing pine-needle oil, fatty acids and caustic soda. Propylene glycol is pure and undiluted CH₃CHOHCH₂OH. Spirits of camphor is an at least 20%-solution of camphor in alcohol, preferably isopropanol. By vinegar essence is meant acetic acid which has been diluted with water to a concentration of 12% acetic acid. The components present in the inventive agent described below have the following functions:

Isopropanol has a bacteria-killing effect; Soap has a cleansing, swelling-reducing and swelling-counteracting effect;
Propylene glycol has a soothing and protecting effect, besides imparting a smoothness to the agent and assisting in maintaining a homogenous mixture;
Spirits of camphor has a swelling-reducing and swelling-counteracting effect and, together with remaining components, renders the agent highly adhesive;
Vinegar essence has a bacteria-killing effect and, together with other components, assists in softening the skin and enhancing the passage of blood therethrough, therewith resulting in reducing and counteracting swellings, among other things;
Cooking salt has a disinfecting effect; and
Water is used to provide an appropriate alcohol content.

The use of these components in skin care treatments is known to the art. The following composition, however, is completely novel and differs from known compositions. Furthermore, the inventive agent has properties and effects which differ greatly from the properties of earlier known agents and the effects achievable therewith.

A preferred composition of the inventive agent is:
40-60% isopropanol
20-40% soft soap
5-10% propylene glycol
2-5% spirits of camphor
1-3% vinegar essence
1-5% cooking salt
10-25% water
These percentages being in percent by volume.

A particularly preferred embodiment of the invention has the following composition:
40% isopropanol
25% soft soap
9% propylene glycol
5% spirits of camphor
2% vinegar essence
4% cooking salt
15% water
The agent is prepared by mixing the components successively together at room temperature in the order given in the above compositions of the recited embodiments. The cooking salt is preferably first dissolved in water and the resultant salt solution then mixed with the vinegar essence, which is finally mixed with the remaining, pre-mixed components. This method of preparation imparts the greatest possible homogeneity to the agent.

## Claims

1. An agent for treating the skin of animals, preferably the skin of livestock or domestic animals where the growth of hair is sparse, and particularly for the treatment of the udders and teats of cows, **characterized** in that the agent comprises a mixture of the following composition, expressed in percent by volume:
40-60% isopropanol
20-40% soft soap
5-10% propylene glycol
2-5% spirits of camphor
1-3% vinegar essence
1-5% cooking salt
10-25% water

2. An agent according to Claim 1, **characterized** in that said agent comprises a mixture of the following composition, expressed in percent by volume:
40% isopropanol
25% soft soap
9% propylene glycol
5% spirits of camphor
2% vinegar essence
4% cooking salt
15% water

3. A method for preparing an agent for the treatment of the skin of animals, preferably the skin of livestock or domestic animals where hair grows sparsely, particularly the udders and teats of cows, said agent comprising a mixture of the following composition, expressed in percent by volume:
40-60% isopropanol
20-40% soft soap
5-10% propylene glycol
2-5% spirits of camphor
1-3% vinegar essence
1-5% cooking salt
10-25% water,
comprising mixing the components at room temperature in the following sequence isopropanol, soft soap, propylene glycol and spirits of camphor, thereafter dissolving the cooking salt in water, mixing the resultant cooking salt solution with vinegar essence, and finally mixing the last mentioned mixture with the earlier mixed components.

4. The non-therapeutical use of an agent for treating the skin of animals with the intention of softening the skin, said agent comprising a mixture of the following composition, expressed in percent by volume:
40-60% isopropanol
20-40% soft soap
5-10% propylene glycol
2-5% spirits of camphor
1-3% vinegar essence
1-5% cooking salt
10-25% water,
comprising applying the agent to essentially hair-free skin on livestock and domestic animals, particularly onto the udders and teats of cows.

## Patentansprüche

1. Mittel zur Behandlung von Tierhaut, vorzugsweise der Haut Von Vieh oder Haustieren, wo der Haarwuchs spärlich ist, und insbesondere für die Behandlung von Eutern oder Zitzen von Kühen,
dadurch **gekennzeichnet,**
daß das Mittel eine Mischung der folgenden Zusammensetzung ausgedrückt in Volumen-% aufweist:
40-60% Isopropanol,
20-40% weiche Seife,
5-10% Propylenglycol,
2-5% Kampferessenzen,
1-3% Essigessenz,
1-5% Kochsalz und
10-25% Wasser.

2. Mittel nach Anspruch 1,
dadurch **gekennzeichnet,**
daß das Mittel eine Mischung der folgenden Zusammensetzung ausgedrückt in Volumen-% aufweist:
40% Isopropanol,
25% weiche Seife,
9% Propylenglycol,
5% Kampferessenzen,
2% Essigessenz,
4% Kochsalz und
15% Wasser.

3. Verfahren zur Zubereitung eines Mittels zur Behandlung von Tierhäuten, vorzugsweise der Haut Von Vieh oder Haustieren, wo der Haarwuchs spärlich ist, insbesondere von Eutern und Zitzen von Kühen, wobei das Mittel eine Mischung der folgenden Zusammensetzung ausgerückt in Volumen-% aufweist:
40-60 % Isopropanol,
20-40 % weiche Seife,
5-10% Propylenglycol,
2-5% Kampferessenzen,
1-3% Essigessenz,
1-5% Kochsalz und
10-25% Wasser,
aufweisend den Schritt des Mischens der Bestandteile bei Raumtemperatur in der folgenden Reihenfolge: Isopropanol, weiche Seife, Propylenglycol und Kampferessenzen, danach Lösen des Kochsalzes im Wasser, Mischen der sich ergebenden Kochsalzlösung mit Essigessenz, und schließlich Mischen der zuletzt genannten Mischung mit den zuvor gemischten Bestandteilen.

4. Nichttherapeutische Verwendung eines Mittels zur Behandlung von Tierhaut zur Erweichung der Haut, wobei das Mittel eine Mischung der folgenden Zusammensetzung ausgedrückt in Volumen-% aufweist:
40-60% Isopropanol,
20-40% weiche Seife,
5-10% Propylenglycol,
2-5% Kampferessenzen,
1-3% Essigessenz,
1-5% Kochsalz und
10-25% Wasser,
aufweisend den Schritt der Aufbringung des Mittels auf im wesentliche haarfreie Haut von Vieh oder Haustieren, insbesondere auf Eutern und Zitzen von Kühen.

## Revendications

1. Agent de traitement de la peau d'animaux, de préférence de la peau de bétail ou d'animaux domestiques aux endroits où la croissance des poils est peu abondante, et particulièrement de traitement des pis et tétons des vaches caractérisé en ce que l'agent comprend un mélange de la composition suivante exprimée en pourcentages en volume :
40-60% d'isopropanol
20-40% de savon doux
5-10% de propylène glycol
2 à 5% d'esprit de camphre
1 à 3% d'essence de vinaigre
1 à 5% de sel de cuisine
10 à 25% d'eau

2. Agent selon la revendication 1 caractérisé en ce que ledit agent comprend un mélange de la composition suivante, exprimée en pourcentages en volume :
40% d'isopropanol
25% de savon doux
9% de propylène glycol
5% d'esprit de camphre
2% d'essence de vinaigre
4% de sel de cuisine
15% d'eau

3. Méthode pour la préparation d'un agent de traitement de la peau d'animaux, de préférence de la peau de bétail ou d'animaux domestiques aux endroits où les poils ont une croissance peu abondante, particulièrement des pis et tétons des vaches, ledit agent comprenant un mélange de la composition suivante exprimée en pourcentages en volume :
40-60% d'isopropanol
20-40% de savon doux
5-10% de propylène glycol
2-5% d'esprit de camphre
1-3% d'essence de vinaigre
1-5% de sel de cuisine
10-25% d'eau,
comprenant le mélange des composants à la température ambiante selon la séquence suivante : isopropanol, savon doux, propylène glycol et esprits de camphre, ensuite la dissolution du sel de cuisine dans l'eau, le mélange de la solution de sel de cuisine résultante avec l'essence de vinaigre et finalement le mélange de la mixture mentionnée en dernier lieu avec les composants préalablement mélangés.

4. Utilisation non thérapeutique d'un agent de traitement de la peau des animaux dans le but d'adoucir la peau, ledit agent comprenant un mélange de la composition suivante, exprimée en pourcentages en volume :
40-60% d'isopropanol
20-40% de savon doux
5-10% de propylène glycol
2-5% d'esprit de camphre
1-3% d'essence de vinaigre
1-5% de sel de cuisine
10-25% d'eau,
comprenant l'application de l'agent sur la peau sensiblement exempte de poils du bétail et des animaux domestiques, notamment sur les pis et les tétons des vaches.
